# EUROPEAN PATENT APPLICATION

(11) **EP 2 138 164 A1**
(43) Date of publication of application: **30.12.2009**
(21) Application number: 09012526.1
(22) Date of filing: 16.06.2006
(51) Int. Cl.: A61K 9/08, A61K 31/337, A61P 35/00

(54) **Liquid pharmaceutical formulations of docetaxel**

(30) Priority: 17.06.2005 AU 2005903196
(62) Divisional of application: 06741249.4
(71) Applicant: Hospira Australia Pty Ltd, Melbourne, VIC 3004 (AU)
(72) Inventor: Liu, Aikun Julie, Endeavour Hills VIC 3802 (AU); Spencer, Allan Harvey, Ferntree Gully VIC 3156 (AU); Knill, Andrew Malcolm, Bitten VIC 3918 (AU); Ash, Daniel David, Hawthron East VIC 3123 (AU)
(74) Representative: Eisenführ, Speiser & Partner

(57) **Abstract**

There is provided a liquid pharmaceutical formulation for parenteral administration comprising:
• docetaxel or a pharmaceutically acceptable salt thereof;
• one or more glycols; and
• a pharmaceutically acceptable nonaqueous solvent system;

wherein the formulation has a pH meter reading in the range of from 2.5 to 7.

## Description

### Field of the invention

The invention relates to liquid pharmaceutical formulations comprising docetaxel that are able to be used as single dose or multi-dose formulations, and to their uses in medicaments and to methods for treating cancer.

### Background of the invention

In this specification, where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

Docetaxel (CAS 114977-28-5) is an antineoplastic agent belonging to the taxoid family which was identified in 1986 as an alternative to paclitaxel. It is prepared by a semi-synthetic process beginning with a precursor extracted from the needles of yew plants (*Taxus baccata*). The chemical name for docetaxel is (2R,3S)-N-carboxy-3-phenylisoserine,N-*tert*-butylester, 13 ester with 5β-20-epoxy-1,2α,4,7β,10β,13α-hexahydroxytax-11-en-9-one 4-acetate 2 benzoate, and it has the following chemical structure:

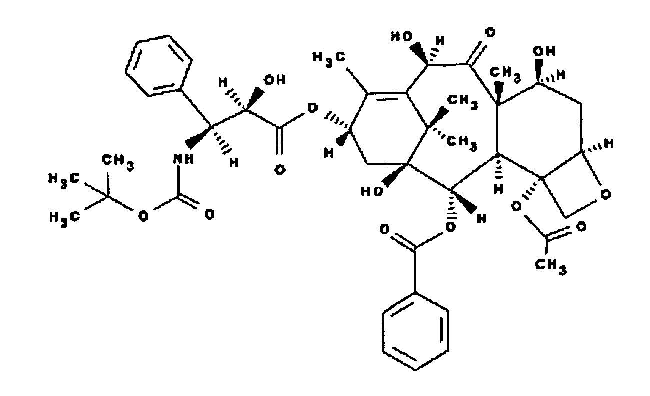

Docetaxel is a white to almost white powder with an empirical formula of C₄₃H₅₃NO₁₄. It is very lipophilic and practically insoluble in water. The first patent family relating to docetaxel includes US patent no 4,814,470 (AU 591,309).

Docetaxel acts by disrupting the microtubular network in cells that is essential for mitotic and interphase cellular functions. Docetaxel binds to free tubulin and promotes the assembly of tubulin into stable microtubules while simultaneously inhibiting their disassembly. This leads to the production of microtubules without normal function and to the stabilization of microtubules which results in the inhibition of mitosis (replication) in cells. Docetaxel's binding to microtubules does not alter the number of protofibrofilaments in the bound microtubules, a feature which differs from most spindle poisons currently in clinical use.

The commercial product marketed by Aventis is called Taxotere^{®} and it was first approved in 1996. It is now approved for a number of different indications throughout the world, as set out below:

| **Indication** | **USA** | **EU** | **AU** | **CA** |
|---|---|---|---|---|
| 2^{nd} line breast cancer | ✔ | ✔ | ✔ | ✔ |
| Breast cancer in combination with capecitabine after anthracycline failure | x | ✔ | ✔ | ✔ |
| Breast cancer: adjuvant treatment of patients with node positive breast cancer in combination with doxorubicin and cyclophosphamide, potentially followed by prophylactic G-CSF | ✔ | ✔ | ✔ | x |
| Breast cancer: combination with doxorubicin with potentially life threatening disease | x | x | x | ✔ |
| Breast cancer: combination with trastuzumab for the treatment of patients with metastatic breast cancer whose tumors overexpress HER2 and who previously have not received chemotherapy for metastatic disease | x | ✔ | x | x |
| 2^{nd} line ovarian cancer | x | x | ✔ | ✔ |
| NSCLC, including those where platinum compound has failed | ✔ | ✔ | ✔ | ✔ |
| NSCLC: combination with cisplatin for the treatment of patients with unresectable, locally advanced or metastatic non-small cell lung cancer who have not previously received chemotherapy for this condition | ✔ | ✔ | x | ✔ |
| Prostate cancer; androgen independent (hormone refractory) | ✔ (in combination with prednisone) | ✔ (in combination with prednisone or prednisolone) | ✔ | x |
| Squamous cell carcinoma of the head and neck; monotherapy or combination after previous failure | x | x | x | ✔ |

As such, it is widely understood that docetaxel is a useful and efficacious oncology agent, either alone or in combination with other agents.

Taxotere^{®} is formulated as a concentrate for dilution. It is a clear-yellow to brownish-yellow viscous solution. Each millilitre contains 40 mg docetaxel and 1040 mg polysorbate 80. The diluent for Taxotere^{®} is 13% ethanol in water for injection. It comes in two presentations:

| **Strength** | **Labelled Fill** | **Diluent (13% ethanol in Water for Injection)** |
|---|---|---|
| Taxotere® 80 | 80 mg docetaxel per 2 ml polysorbate 80 | 6 ml |
| Taxotere® 20 | 20 mg docetaxel per 0.5 ml polysorbate 80 | 1.5 ml |

The medical practitioner must aseptically withdraw the entire contents of the diluent vial, transfer it to the vial containing the docetaxel concentrate and mix the components to produce a solution containing 10 mg/ml docetaxel. That mixture must be repeatedly inverted for 45 seconds in order to mix the solutions adequately. It cannot be shaken, as that leads to foaming and the potential loss of potency. This intermediate solution is then diluted in an infusion bag, typically 250 ml, containing either 0.9% sodium chloride solution or 5% dextrose solution to produce a concentration of 0.3 to 0.74 mg/ml of docetaxel.

Due to the fact that docetaxel is practically insoluble in water, there have been a number of other attempts to develop appropriate injectable formulations. For example, docetaxel is known to be soluble in ethanol and one of the first such other formulations was 50% ethanol and 50% Emulphor EL^{®} (a non-ionic solubilizer and emulsifier manufactured by reacting castor oil with ethylene oxide).

US patent no 5,403,858 (AU 666,859; EP593 601; EP522 937) discloses a formulation for docetaxel which reduces the ethanol concentration, or eliminates the ethanol from the solution completely. The formulations comprise a surfactant, such as a polysorbate (eg Tween^{®}), a polyoxyethylene glycol ester (eg. Emulphor^{®}) or an ester of polyethylene glycol and castor oil (eg Cremophor EL^{®}); and are virtually free from ethanol.

US patent no 5,714,512 is also part of this patent family and relates to formulations consisting essentially of docetaxel dissolved in a surfactant selected from polysorbate, polyoxyethylated vegetable oil and polyethoxylated castor oil which are essentially free of ethanol. US patent no 5,698,582 is also part of this patent family and relates to formulations comprising docetaxel dissolved in a surfactant selected from polysorbate or polyethoxylated castor oil which is essentially free of ethanol.

AU691476 (EP 0 671 912) discloses a two part injectable composition. This two part composition involves preparing an intermediate solution using the stock solution, prior to the addition of this intermediate solution to infusion bag. The intermediate solution contains an additive which promotes the dissolution of the stock solution in the aqueous infusion solution by breaking or avoiding the formation of a gelled phase between the surfactant in the stock solution and the water of the infusion solution. The additives have a molecular weight equal to or less than 200 and have at least one hydroxyl functional group or one amine functional group, for example, ethanol, glucose, glycerol, propylene glycol, glycine, sorbitol, mannitol, benzyl alcohol and polyethylene glycols. The additives may also be inorganic salts such as sodium chloride.

There are a number of other patent applications which have been made for formulations of docetaxel. However, none of these attempted formulations has resulted in a successful commercial product to compete with Taxotere^{®} to date. There is thus still a need for alternative docetaxel formulations which have the necessary physicochemical properties, bioavailability and shelf life.

One of the difficulties with the currently commercially available formulation of docetaxel, Taxotere^{®}, is that the administration process is complex and involves many steps. As described above, the person administering the drug must first create an intermediate solution before then administering that intermediate solution into the infusion bag. As docetaxel is extremely toxic, all steps should be taken to minimise the handling that is required in administering the drug.

In making that intermediate solution, the medical practitioner must manually invert the vial for 45 seconds. The prescribing information for Taxotere^{®} gives very clear instructions not to shake the vial. This is due to the foaming that can occur, potentially resulting in potency loss.

A further difficulty of the Taxotere® product is that the intermediate solution must be added to the infusion bag within 8 hours of making that admixture. Accordingly, the current commercially available presentation of docetaxel is a single use vial only.

Further, once added to the infusion bag, it has a limited stability in the infusion bag. The prescribing information for Taxotere® states it is only stable for four hours and must be used within this period.

### Summary of the invention

It has surprisingly been found that a docetaxel formulation comprising the combination of pH modification and a glycol in a non-aqueous solvent has the following advantages:
(a) comparative stability of the formulation when compared to the Taxotere^{®} concentrate (ie pre-dilution);
(b) suitable for use as a multi-dose product due to the increased alcohol content;
(c) it is a single vial product ready for introduction directly into the infusion bag without the need for any intermediate solution, therefore requiring less handling by the medical practitioner prior to administration to a patient;
(d) more accurate dosage of the drug as a consequence of the reduced foaming when preparing the product minimising the risk of potency loss; and
(e) comparative stability once the formulation is introduced to the infusion solution.

According to a first aspect of the invention, there is provided a liquid pharmaceutical formulation for parenteral administration comprising:
- docetaxel or a pharmaceutically acceptable salt thereof;
- one or more glycols; and
- a pharmaceutically acceptable nonaqueous solvent system;
wherein the formulation has a pH meter reading in the range of from 2.5 to 7.

According to a second aspect of the invention, there is provided a pharmaceutical liquid formulation for parenteral administration comprising:
- docetaxel or a pharmaceutically acceptable salt thereof;
- one or more glycols;
- an amount of one or more pharmaceutically acceptable acids sufficient to provide the formulation with a pH meter reading in the range of from 2.5 to 7; and
- a pharmaceutically acceptable nonaqueous solvent system.

A person skilled in the art will know that pH is a measure of free H⁺ ions in a solution. For example, free H⁺ will exist in alcohol systems which contain acids. The pH may be measured by placing a pH meter directly into the liquid formulation, such pH meter having been calibrated for the appropriate pH range with standard aqueous buffers. Persons skilled in the art will know of other methods which may be used to measure pH. Such a person will further know that, while the pH meter reading obtained for a substantially non-aqueous formulation may not be a true reflection of the actual H⁺ ion concentration in the solution, it may nonetheless give a meaningful and reproducible measurement that indicates the relative acidity/basicity of the solution as is the case for the docetaxel formulations disclosed herein. Preferably, the pH meter reading is in the range from 3 to 7, more preferably 3 to 6. Most preferably, the pH meter reading is in the range of from 4 to 6. These ranges are for measurements made at room temperature (20 to 25°C). A person skilled in the art will know that the pH meter reading will vary depending on the temperature.

The pH of a formulation comprising 10mg docetaxel, 260 mg polysorbate 80, 0.23 ml ethanol and PEG 300 to one ml had a pH reading of 8.2. Polysorbate 80 on its own had a pH reading of 8.

A person skilled in the art will recognise that the pH meter reading of the formulations according to the invention can be achieved by acidifying the formulation itself, or by adjustment of the pH of any of the components of the formulation, for example by purification of the surfactant to remove basic contaminants or acidification of any one of the components prior to the mixing of the formulation.

The acid may be selected from the range of pharmaceutically acceptable acids known to those skilled in the art, which are soluble in the nonaqueous solvent system and which are compatible with docetaxel. A person skilled in the art will know that certain strong acids may react with docetaxel creating degradants and to avoid such acids. For example, epimerisation of the hydroxyl functionality of docetaxel is known to be facilitated by certain strong acids. In some instances, the use of a stabilising agent may counteract any degradative effect of the acid. The acid may be inorganic or organic. Preferably, the pharmaceutically acceptable acids are organic acids. More preferably, the pharmaceutically acceptable acid is selected from carboxylic and dicarboxylic acids. Most preferably, the pharmaceutically acceptable acid is selected from citric acid, tartaric acid, acetic acid and mixtures thereof.

A person skilled in the art will know that the amount of pharmaceutically acceptable acid used will be limited by the particular acid's solubility in the pharmaceutically acceptable nonaqueous solvent system. The amount of acid required will also be further determined by the relative strength of the acid.

Where the pharmaceutically acceptable acid is citric acid, then preferably the citric acid is present at a concentration in the range of from 1.6 to 6 mg/ml, more preferably 4 mg/ml.

The docetaxel used to make the formulation of the invention may be in any form known to those skilled in the art including anhydrous forms, hydrated forms, polymorphs, derivatives and pro-drugs.

The concentration of docetaxel may be any amount up to 90 mg/ml. Preferably, the concentration of docetaxel is in the range of from 5 to 20 mg/ml, more preferably from 8 to 12 mg/ml, and most preferably about 10 mg/ml.

The glycol is preferably selected from the group consisting of polyethylene glycols, propylene glycol, tetra glycol and mixtures thereof. Polyethylene glycol (eg PEG 300 and PEG 400) is an excipient which is widely used in pharmaceutical formulations. Preferably, the polyethylene glycol has a molecular weight in the range from 200 to 600. More preferably, the polyethylene glycol has a molecular weight of about 300 (PEG 300). A person skilled in the art will know that a polyethylene glycol having a molecular weight above 600 is likely to be solid and can be used in nonaqueous systems.

Propylene glycol and tetra glycol are also used in pharmaceutical formulations as solvents and are approved for parenteral use by the regulatory authorities around the world, including the US Food and Drug Administration and the equivalent European authority.

Preferably, the glycol is present in the formulation in an amount in the range of from 30 to 65% v/v, more preferably about 57%.

The pharmaceutically acceptable nonaqueous solvent system may comprise any pharmaceutically acceptable nonaqueous components known to persons skilled in the art in which the docetaxel is soluble; for example, alcohols and surfactants. Typically, the pharmaceutically acceptable nonaqueous solvent system will comprise one or more alcohols; and one or more non-ionic surfactants selected from the group consisting of polyethoxylene sorbitan fatty acid esters (polysorbates) such as Tween 80^{®}, polyoxyethylene glycol esters such as Emulphor^{®}, and polyethoxylated castor oils such as Cremophor-EL^{®} and mixtures thereof. Preferably, the alcohol is ethanol and the surfactant is a polysorbate.

Preferably, the alcohol is present in an amount in the range of from 10 to 55% v/v of the formulation, more preferably 18 to 26%, and most preferably about 23% v/v.

Preferably, the non-ionic surfactant is present in an amount in the range of from 10 to 50% v/v of the formulation, more preferably 10 to 40%, and most preferably about 25%.

The pharmaceutically acceptable nonaqueous solvent system may include other components such as a solubilising agent, eg benzyl benzoate, or stabilising agents, eg povidone.

The person skilled in the art will understand that whilst the solvent system is described as nonaqueous, this merely indicates that water is not specifically added to the formulation. There is likely to be some water present in the formulation due to its presence in some of the commercial components used (eg surfactants) and water may also be absorbed from the environment into the formulation. Formulations containing these incidental amounts of water are included within the scope of the invention.

A person skilled in the art preparing formulations according the invention will understand that the proportion of components with respect to each other will vary depending on the specific components used. For example, the use of different surfactants and alcohols will require some straightforward modifications to the proportions depending on the miscibility of a particular surfactant in a particular alcohol. A skilled person will understand that the appropriate relative ratios of each of the excipients have been obtained when a homogeneous solution results from the admixture of all ingredients, and the docetaxel remains in solution.

The pharmaceutical formulation will typically comply with the *International Conference on Harmonisation (ICH) Guidelines.*

In a preferred embodiment, there is provided a liquid formulation for parenteral administration comprising:
- docetaxel or a pharmaceutically acceptable salt thereof at a concentration in the range of from 5 to 20 mg/ml;
- one or more polyethylene glycols in an amount in the range of from 30 to 65% v/v;
- one or more pharmaceutically acceptable acids in an amount sufficient to provide the formulation with a pH meter reading in the range of from 3 to 7;
- one or more alcohols in an amount in the range of from 10 to 55% v/v; and
- one or more surfactants in an amount in the range of from 10 to 50% v/v.

In one preferred embodiment, the pharmaceutical liquid formulation for parenteral administration comprises:
- docetaxel at a concentration in the range of from 6 to 20 mg/ml;
- polyethylene glycol 300 in an amount in the range of from 30% to 65% v/v;
- citric acid at a concentration in the range of from 1.6 to 6 mg/ml;
- polysorbate 80 in an amount in the range of from 10 to 55% v/v; and
- ethanol in an amount in the range of from 10 to 50% v/v.

In a particularly preferred embodiment, the pharmaceutical liquid formulation for parenteral administration comprises:
- a concentration of about 10 mg/ml docetaxel;
- about 57% v/v of polyethylene glycol 300;
- a concentration of about 4 mg/ml of citric acid;
- about 25% v/v of polysorbate 80; and
- about 23% v/v of ethanol.

In another preferred embodiment, the pharmaceutical liquid formulation for parenteral administration comprises:
- docetaxel at a concentration in the range of from 6 to 20 mg/ml;
- citric acid at a concentration in the range of from 1.6 to 6 mg/ml;
- polysorbate 80 in an amount in the range of from 10 to 55% v/v;
- ethanol in an amount in the range of from 10 to 50% v/v; and
- polyethylene glycol 300 in an amount sufficient to make up the formulation to QS 100%

In another particularly preferred embodiment, the pharmaceutical liquid formulation for parenteral administration comprises:
- a concentration of about 10 mg/ml docetaxel;
- a concentration of about 4 mg/ml of citric acid;
- about 25% v/v of polysorbate 80;
- about 23% v/v of ethanol; and
- polyethylene glycol 300 in an amount sufficient to make up the formulation to QS 100%

According to a third aspect of the invention, there is provided the use of a pharmaceutical formulation according to the first and second aspects in the preparation of a medicament for the treatment of a cancer.

According to a fourth aspect of the invention, there is provided a method for treating a cancer which comprises administering a pharmaceutical formulation according to the first and second aspects to a patient in need thereof.

According to a fifth aspect of the invention, there is provided an infusion solution produced by the admixture of a pharmaceutical formulation according to the first and second aspects of the invention and an infusion diluent, typically 0.9% NaCl or 5% dextrose or glucose.

### Drawings

Various embodiments/aspects of the invention will now be described with reference to the following drawings in which:
Table 1 shows the impurity profile of the formulations in Example 1 at the initial time point.
Table 2 shows the impurity profile of the formulations in Example 1 at one month.
Table 3 shows the impurity profile of the formulations in Example 1 at two months.
Table 4 shows the impurity profile of Formulation 3 in Example 1 at 3, 4 and 5 months.
Table 5 shows the impurity profile of the formulations in Example 2 at the initial time point.
Table 6 shows the impurity profile of the formulations in Example 2 at one month.
Table 7 shows the impurity profile of the formulations in Example 3.
Table 8 shows the impurity profile of the formulations in Example 4.
Table 9 shows the impurity profile of the formulations in Example 5.
Table 10 shows the impurity profile of the formulations in Example 6.
Table 11 shows the impurity profile of the formulations in Example 7.
Table 12 shows the impurity profile of the formulations in Example 8.
Table 13 shows the results obtained for NaCl solution in Example 9.
Table 14 shows the results obtained for glucose solution in Example 9.

In these tables, the level of impurities is provided as % peak area.

The following abbreviations are used in the tables.

| | |
|---|---|
| ND = not detected | n/t = not tested |
| N/R= not recorded as peak areas <0.05% n/a= not applicable | n/t = not tested |

### Examples

Various aspects of the invention will now be described with reference to the following nonlimiting examples.

### Components used in formulations

All components including the docetaxel were standard pharmaceutical grade quality.

Polyethylene glycols are widely used in a variety of pharmaceutical formulations including parenteral, topical, ophthalmic, oral, and rectal preparations. Polyethylene glycols are stable, hydrophilic substances that are essentially non-irritant to the skin. Although they do not readily penetrate the skin, polyethylene glycols are water soluble and as such are easily removed from the skin by washing; they are therefore useful as ointment bases. Solid grades are generally employed in topical ointments with the consistency of the base being adjusted by the addition of liquid grades of polyethylene glycol.

Propylene glycol is used as an antimicrobial preservative; disinfectant; humectant; plasticizer; solvent; stabilizer for vitamins; and water-miscible cosolvent. Propylene glycol has become widely used as a solvent, extractant, and preservative in a variety of parenteral and nonparenteral pharmaceutical formulations. It is a better general solvent than glycerin and dissolves a wide variety of materials, such as corticosteroids, phenols, sulfa drugs, barbiturates, vitamins (A and D), most alkaloids, and many local anaesthetics.

Citric acid, as either the monohydrate or anhydrous material, is widely used in pharmaceutical formulations and food products primarily to adjust the pH of solutions. Citric acid monohydrate is used in the preparation of effervescent granules while anhydrous citric acid is widely used in the preparation of effervescent tablets.

Tartaric acid is used in beverages, confectionery, food products, and pharmaceutical formulations as an acidulant. It may also be used as an acidifying agent, a sequestering agent, and as an antioxidant synergist. In pharmaceutical formulations, it is widely used in combination with bicarbonates, as the acid component of effervescent granules, powders, and tablets.

Polyethoxylene sorbitan fatty acid esters (polysorbates) are a series of partial fatty acid esters of sorbitol and its anhydrides co-polymerized with approximately 20, 5 or 4 moles of ethylene oxide for each mole of sorbitol and its anhydrides. The resulting product is a mixture of molecules of different sizes. Polysorbates are used as solubilising agents for a variety of substances including oil-soluble vitamins and as wetting agents in the formulation of oral and parenteral suspensions. Polysorbate 80 is approved by the FDA, EMEA and TGA for parenteral use.

Ethanol is commonly used as a solvent, anti-microbial preservative, disinfectant and penetration enhancer. Ethanol and aqueous ethanol solutions of various concentrations are widely used in pharmaceutical formulations and cosmetics. Although ethanol is primarily used as a solvent it is also employed in solutions as an antimicrobial preservative.

Benzyl benzoate is used as a plasticizer; solubilising agent; solvent; and therapeutic agent. Benzyl benzoate is used as a solubilising agent and nonaqueous solvent in intramuscular injections at concentrations between 0.01 to 46.0% v/v. It is also used as a solvent and plasticizer for cellulose and nitrocellulose. However, the most widespread pharmaceutical use of benzyl benzoate is as a topical therapeutic agent in the treatment of scabies.

### Formulations

All formulations referred to in the following examples have been prepared using the following mixing process.
- Add required amount of ethanol into a clean, dry beaker mixing vessel.
- Add acid into the vessel containing absolute alcohol. Mix until all dissolved.
- Add docetaxel active ingredient and mix until solution becomes clear.
- Add polysorbate 80 (pre-flushed with nitrogen) into above solution, mixed until solution becomes homogeneous.
- Make up the solution to final volume using PEG 300.
- Mixed and flushed with nitrogen for at least 10 minutes.
- Close and seal the solution and kept at room temperature until filtration and filling.
- Filter the bulk solution through a suitable sterile filter.
- Fill the solution into a clear type I glass vial, and capped with a rubber stopper that is suitable for parenteral and compatible with docetaxel solution.

Two stoppers and 1 type of clear Type 1 glass vials were tested, and were found satisfactory. A person skilled would know to avoid stoppers and vials that were subject to materials being extracted from the stopper and vials by the formulation components contained therein.

### Methods

Each of the formulations prepared was subjected to accelerated stability testing at 40°C.

### pH measurement

The pH reading was taken using a standard laboratory pH meter and method. The pH meter used was a pH 330 pocket pH/mV meter with electrode model SenTix 81, both of which are manufactured by WTW. The pH meter was calibrated using standard aqueous buffers at pH 7.0 and 3.0. The pH meter electrode was inserted directly into the undiluted solution. After the initial fluctuation in the reading resolved, the pH meter reading was taken. A person skilled in the art would recognise that there is some fluctuation in the initial reading of a pH meter with both aqueous and non-aqueous solutions, but that the reading will resolve and stabilise in a period of time between 30 seconds and 5 minutes, typically one minute. Whilst the fluctuation may be greater in a non-aqueous system, stabilisation does still occur.

### Impurity analysis

The analysis of the impurities was undertaken using reverse phase High Performance Liquid Chromatography (HPLC). HPLC is a technique that is widely used and well known in the art. HPLC can be used to measure the potency of the docetaxel where potency is defined as a percentage of the initial concentration of docetaxel. HPLC can also be used to measure the relative proportions of known and unknown impurities in a docetaxel formulation. Any suitable HPLC method which will separate the impurities may be used.

Impurity levels were calculated by peak area normalisation.

### Example 1

The following formulations were prepared.

| **Materials** | **F1** | **F2** | **F3** | **F4** |
|---|---|---|---|---|
| Docetaxel | 10 mg | 10 mg | 10 mg | 10 mg |
| Polysorbate 80 | 520 mg | 260 mg | 260 mg | 260 mg |
| Citric acid | n/a | 2 mg | 1.6 mg | n/a |
| Ethanol (absolute) | qs to 1.0 ml | qs to 1.0 ml | 0.23 ml | 0.25 ml |
| PEG 300 | n/a | n/a | qs to 1 ml | qs to 1 ml |

Formulation F1 replicates the formulation which was used in the docetaxel clinical trials by Aventis. Formulation F2 contains an acid but no PEG 300. Formulation F4 contains PEG 300 but no acid. Formulation F3 contains both acid and PEG 300.

### Control Formulations

Taxotere^{®} 20 (Aventis, B/No: 4 D404/4B057, Expiry: 10/2005) was tested as the control. The product as purchased commercially was tested, that is, the two vial system was subjected to the accelerated stability trials. However, the two vials of the Taxotere were only combined at the time of testing the sample for pH measurement and colour. The potency and impurities described in this example were determined using the storage form of Taxotere^{®}, namely the single vial containing the docetaxel prior to the combining of the two vials.

### Results & discussion

At the initial time point (Table 1), Formulation F3 did not produce any significant impurities when compared with the unformulated docetaxel active ingredient which had been stored at 2 to 8°C. Formulation F3 was observed to have less impurities than Taxotere^{®} 20.

From the results at one month (Table 2), it is clear that formulation F3 was significantly more stable than formulation F2 and the key difference between these two formulations was the PEG 300. This indicates that PEG 300 has a stabilising effect on docetaxel. However, it is apparent from the results for formulation F4 that the use of PEG 300 alone is not sufficient to reduce the level of impurities to a level that would be satisfactory for a commercial pharmaceutical formulation. These results show plainly that the combination of PEG 300, polysorbate 80, alcohol and acidification leads to a more stable docetaxel formulation.

From the results at the one month time point, it was decided to only continue with Formulation F3. The results at two months (Table 3) show that Formulation F3 has a lower level of total impurities than that of the Taxotere^{®} 20 control.

The results for Formulation F3 at 3, 4 and 5 months is shown in Table 4.

In summary, the impurity results indicate that formulation F3 was observed to have at least comparative stability with the Taxotere^{®} 20 presentation.

### Example 2

In this example, further formulations according to the invention were tested.

The following formulations were prepared.

| **Formulation** | **Composition** |
|---|---|
| F5 | 10 mg docetaxel, 260 mg polysorbate 80, 2.0 mg citric acid, 0.23 ml ethanol (absolute) and PEG 300 QS to 1 ml. Filled under nitrogen. |
| F6 | 10 mg docetaxel, 260 mg polysorbate 80, 2.0 mg citric acid, 0.20 ml ethanol (absolute) and PEG 300 QS to 1 ml. |
| F7 | 10 mg docetaxel, 260 mg polysorbate 80, 4.0 mg citric acid, 0.20 ml ethanol (absolute) and PEG 300 QS to 1 ml. |
| F8 | 10 mg docetaxel, 260 mg polysorbate 80, 6.0 mg citric acid, 0.20 ml ethanol (absolute) and PEG 300 QS to 1 ml. |
| F9 | 10 mg docetaxel, 260 mg polysorbate 80, 2.0 mg citric acid, 0.25 ml ethanol (absolute) and PEG 300 QS to 1 ml. |
| F10 | 10 mg docetaxel, 520 mg polysorbate 80, 2.0 mg citric acid, 0.10 ml ethanol (absolute) and PEG 300 QS to 1 ml. |
| F11 | 10 mg docetaxel, 260 mg polysorbate 80, 2.0 mg tartaric acid, 0.20 ml ethanol (absolute) and PEG 300 QS to 1 ml. |
| F12 | 20 mg docetaxel, 260 mg polysorbate 80, 2.0 mg citric acid, 0.20 ml ethanol (absolute) and PEG 300 QS to 1 ml. |
| F13 | 20 mg docetaxel, 520 mg polysorbate 80, 2.0 mg citric acid, 0.20 ml ethanol (absolute) and PEG 300 QS to 1 ml. |
| F14 | 10 mg docetaxel, 520 mg polysorbate 80, 2.0 mg citric acid, 0.10 ml ethanol (absolute) and PEG 300 QS to 1 ml. |

The formulations were subjected to accelerated stability trials and the pH, potency and impurities were tested as per Example 1.

### Results and discussion

The initial impurity profile is shown in Table 5 with the results at one month in Table 6. There was not enough sample remaining at one month to take the pH measurement so the pH at 2 months is provided.

The results show that formulations according to the invention with varying amounts of the docetaxel, acid, ethanol and polysorbate or with different acids are stable.

### Example 3

This example investigated the stability of formulations according to the invention which contain different glycols.

The following formulations were prepared and potency assay and related substances compared at time 0 and 1 month for 25 and 40°C.

| **Formulation** | **Composition** |
|---|---|
| C1 | 10 mg docetaxel, 260 mg polysorbate 80, 4.0 mg citric acid, 0.23 ml ethanol and PEG-300 QS to 1 ml |
| F15 | 10 mg docetaxel, 260 mg polysorbate 80, 4.0 mg citric acid, 0.23 ml ethanol and propylene glycol QS to 1 ml |
| F16 | 10 mg docetaxel, 260 mg polysorbate 80, 4.0 mg citric acid, 0.23 ml ethanol and tetra glycol QS to 1 ml |

### Results and discussion

The results are in Table 7. The impurity profile for all F16 T = 0 and 1 month samples look nearly identical and within experimental error. Interestingly, in contrast to C1, the amounts of some impurities in F16 do not increase under the accelerated stability conditions.

For F17, only very minor known and unknown impurities appear in the impurity profile as the stability experiment progressed.

These results clearly show that a range of different glycols can be used in the formulation according to the invention. It would therefore be understood by the person skilled in the art that other glycols are readily substitutable in the invention.

### Example 4

This example investigated the stability of formulations according to the invention which contain different pharmaceutically acceptable acids.

The following formulations were prepared and potency assay and related substances compared at time 0 and 1 month for 25 and 40°C.

The pH adjustment for F18 was made by reference to the pH of acidified ethanol with citric acid. The pH reading of the citric acid acidified ethanol used in C 1 was recorded following its addition to docetaxel. For F18, sufficient acetic acid was added to obtain that pH reading obtained for C1 after the addition of the 4.0mg of citric acid and the 10 mg of docetaxel to the ethanol.

| **Formulation** | **Composition** |
|---|---|
| C1 | 10 mg docetaxel, 260 mg polysorbate 80, 4.0 mg citric acid, 0.23 ml ethanol and PEG-300 QS to 1 ml |
| F17 | 10 mg docetaxel, 260 mg polysorbate 80, 0.23 ml ethanol pH adjusted using acetic acid followed by addition of PEG-300 QS to 1 ml |

### Results and discussion

The results are in Table 8. Acetic acid in F 18 causes minor increases in known and unknown impurities in this formulation when compared to the control, but is within the range of what would be considered pharmaceutically acceptable stability.

When combined with the results for the tartaric acid seen above in Example 2 (formulation F11), these results clearly show that different organic acids can be used in the formulation according to the invention.

### Example 5

This example investigated the stability of formulations according to the invention which contain different non-ionic surfactants in the nonaqueous solvent system. As noted previously, a person skilled in the art will recognise that the use of different components, including a different surfactant, may require adjustments to be made to the relative ratios of the components of the formulation.

The following formulations were prepared and potency assay and related substances compared at time 0 and 1 week for 25 and 40°C.

| **Formulation** | **Composition** |
|---|---|
| C1 | 10 mg docetaxel, 260 mg polysorbate 80, 4.0 mg citric acid, 0.23 ml ethanol and PEG-300 QS to 1 ml |
| F18 | 10 mg docetaxel, 315 mg Cremophor®, 5.2 mg citric acid, 0.3 mL ethanol and PEG-300 QS to 1 mL |

### Results and discussion

The results are in Table 9.

These results clearly show that different non-ionic surfactants in a suitable non-aqueous solvent system vehicle can be used in the formulation according to the invention.

### Example 6

This example demonstrates that other pharmaceutically acceptable excipients may be included within the formulation according to the invention.

The following formulations were prepared and potency assay and related substances compared at time 0 and 1 month for 25 and 40°C.

| **Formulation** | **Composition** |
|---|---|
| C1 | 10 mg docetaxel, 260 mg polysorbate 80, 4.0 mg citric acid, 0.23 ml ethanol and PEG-300 QS to 1 ml |
| F19 | 10 mg docetaxel, 260 mg polysorbate 80, 4.0 mg citric acid, 4.0 mg povidone 12F, 0.23 ml ethanol and PEG-300 QS to 1 ml |

### Results and discussion

The results are in Table 10. F19 has a similar stability profile to that observed for the control C1.

These results clearly show that other stabilising agents (eg povidone) can be used in a suitable nonaqueous solvent system in the formulation according to the invention.

### Example 7

This example demonstrates that other pharmaceutically acceptable solvents may be included within the formulation according to the invention.

The following formulations were prepared and potency assay and related substances compared at time 0 and 1 month for 25 and 40°C.

| **Formulation** | **Composition** |
|---|---|
| C1 | 10 mg docetaxel, 260 mg polysorbate 80, 4.0 mg citric acid, 0.23 ml ethanol and PEG-300 QS to 1 ml |
| F20 | 10 mg docetaxel, 260 mg polysorbate 80, 4.0 mg citric acid, 0.10 ml ethanol, 0.13 benzyl benzoate and PEG-300 QS to 1 ml |

### Results and discussion

The results are in Table 11. The impurity profile for F20 is consistent as time and temperature increases. The major impurity at RRT = 0.9 (>0.5%) is believed to be associated with the excipient benzyl benzoate and not a degradation product of docetaxel.

These results clearly show that other solvents can be used in a suitable nonaqueous solvent system in the formulation according to the invention.

### Example 8

The following formula according to the invention was prepared and its stability investigated.

| **Component** | **Amount** |
|---|---|
| Docetaxel | 10.67 mg |
| Polysorbate 80 | 260 mg |
| Citric Acid | 4 mg |
| Absolute alcohol (ethanol) | 0.23 ml |
| PEG 300 | qs to 1 ml |
| Headspace | Nitrogen |

### Results

The results obtained after storage at 25°C and 40°C for 15 weeks are in Table 12.

### Example 9

This example investigated the stability of formulations according to the invention when diluted in infusion bags as they would be prior to administration.

The formulation according to the invention used in this example is as follows:

| **Component** | **Formula** |
|---|---|
| Docetaxel (anhydrous) | 10 mg |
| Polysorbate 80 | 260 mg |
| Citric Acid | 4 mg |
| Absolute alcohol | 0.23 ml |
| PEG 300 qs to | 1 ml |
| Headspace | Nitrogen |

As a control, infusion bags containing the current commercial product Taxotere^{®} were also prepared. The infusion bags were prepared according to the Taxotere^{®} instructions for both the Taxotere^{®} and the formulation according to the invention to produce a solution having a final concentration of docetaxel of 0.74 mg/ml. Infusion bags were prepared using both 0.9% NaCl solution and 5% glucose solution.

The infusion bags were analysed for clarity, particulates and chemical stability.

### Results

Table 13 includes the results obtained for the 0.9% NaCl solution, where:
N- clear colourless solution free from visible matter
N*-Slightly cloudy solution, no visible matter observed
N**- Cloudy solution, visible matter observed

Table 14 includes the results obtained for the 5% glucose solution, where:
N- clear colourless solution free from visible matter
N*-Slightly cloudy solution, no visible matter observed
N**- Cloudy solution, visible matter observed

"OOS" in these tables indicates the measurement was "outside of specification", that is, no longer considered suitable for administration.

"Particulates complies" in these tables indicates that the formulation complies with the Particulates Test Acceptance Criteria (USP/BP/Ph.Eur requirement).

| **Fill Volume** | **Particle Sizes** | **Acceptance Criteria** |
|---|---|---|
| Small Volume Injections | = 10 um | < 6000 counts /container |
| (< 100mL ) | = 20 um | < 600 counts /container |

### Discussion

For an infusion bag solution to be considered stable and suitable for use, it must remain a clear, colourless solution free from visible matter and particulates. If the solution becomes cloudy, it is no longer suitable for use, particularly where an in-line filter is used during administration.

From the results generated with 0.9% sodium chloride bags, Taxotere^{®} was observed to be stable in the bag for up to four hours. The formulation according to the invention was observed to be stable for at least four hours.

From the results generated using the 5% glucose bags, the formulation according to the invention was clear and colourless up to 6 hours. The particulates test for the formulation according to the invention also complied with stability requirements for up to six hours. Unfortunately due to lack of sample, the formulation according to the invention could not be tested for particulates at six hours as approximately 25 ml of sample is required for each test point. At 7.5 hours, the formulation according the invention's physical stability seemed to change dramatically wherein the appearance of solution was seen to be cloudy with visible matter observed and therefore all other associated testing was not carried out.

From the results generated using the 5% glucose bags, Taxotere^{®} was found to go cloudy at four hours with particulate counts 25µm higher at three hours (6398) compared to the formulation according to the invention at four hours (633). The pH did not change between testing initially to five hours, however, no other testing was carried out at five hours due to the cloudiness of the Taxotere^{®} solution indicating instability.

### Conclusion

From this study, it can be concluded that the formulation according to the invention is stable for at least four hours in the NaCl infusion bag and at least six hours in the glucose bag. Based on these results, therefore, it can be concluded that the formulation according to the invention is at least as physically stable as Taxotere^{®}in the infusion bag, and appears to have improved stability (particularly in a glucose solution).

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

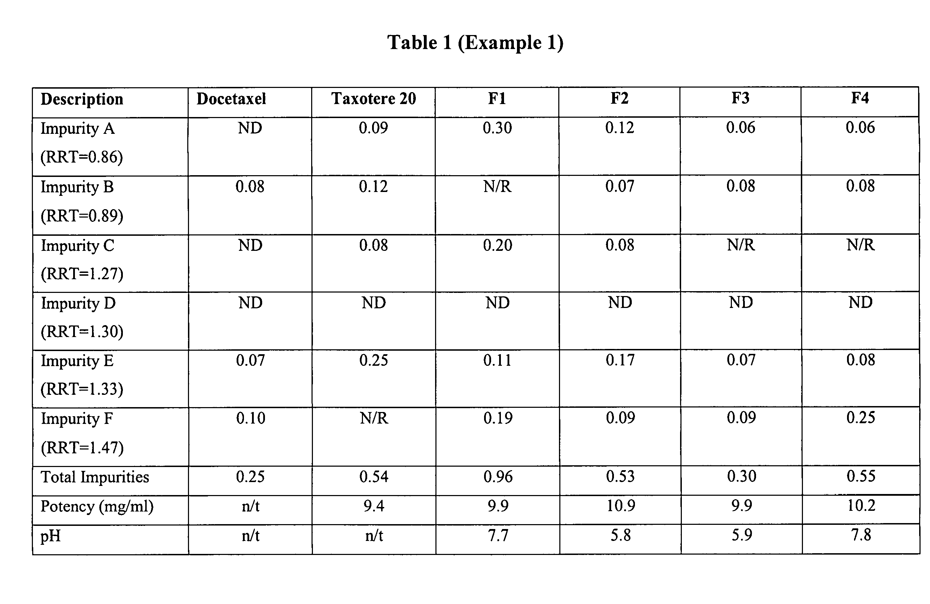

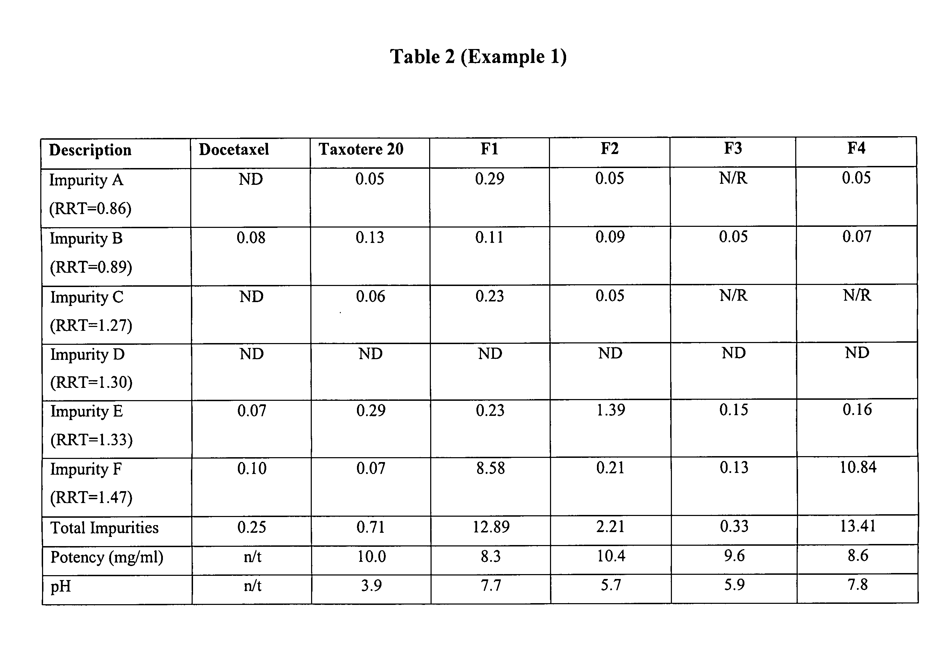

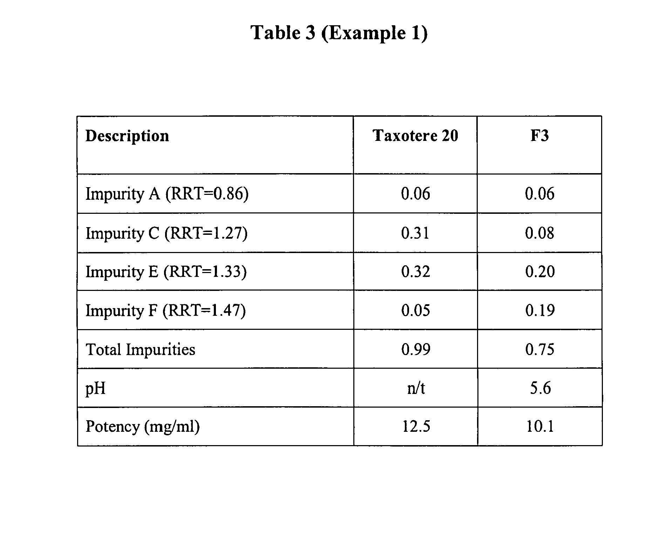

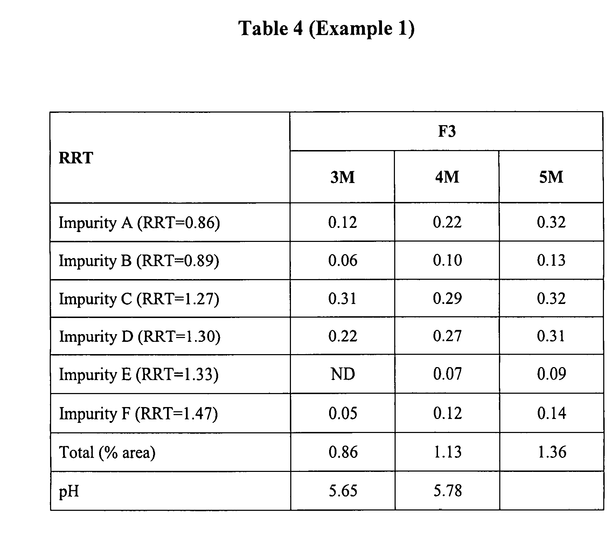

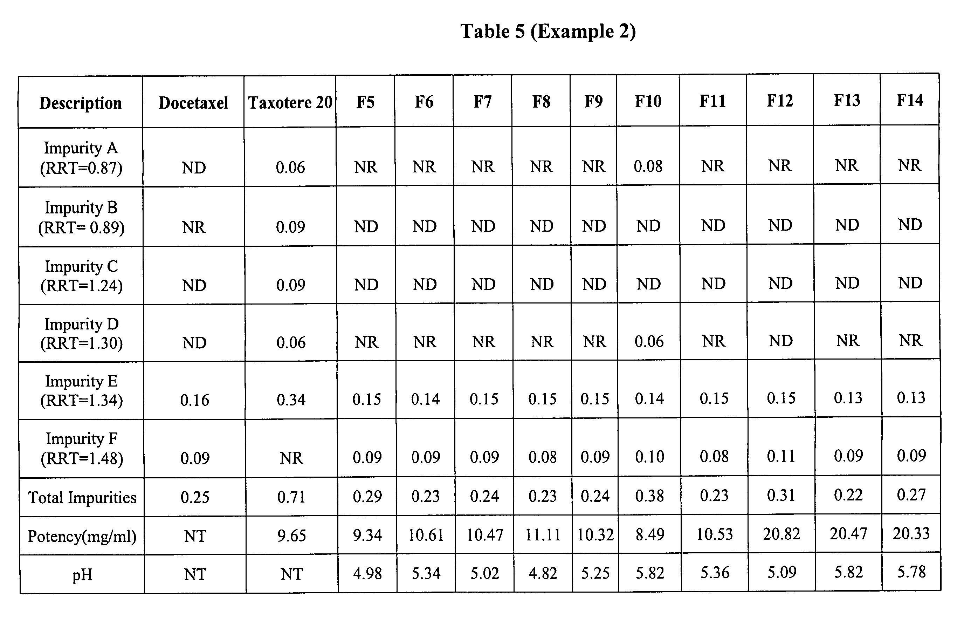

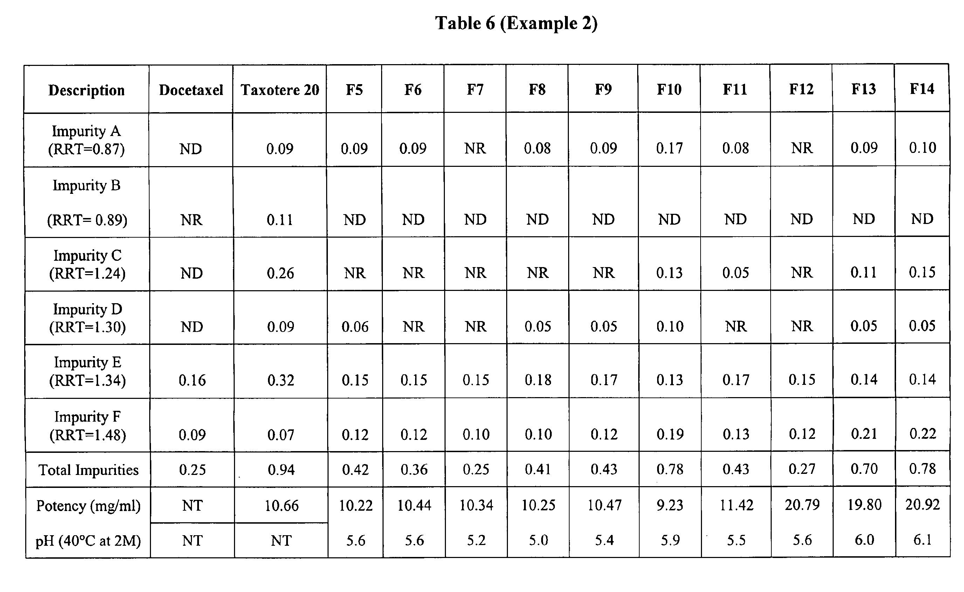

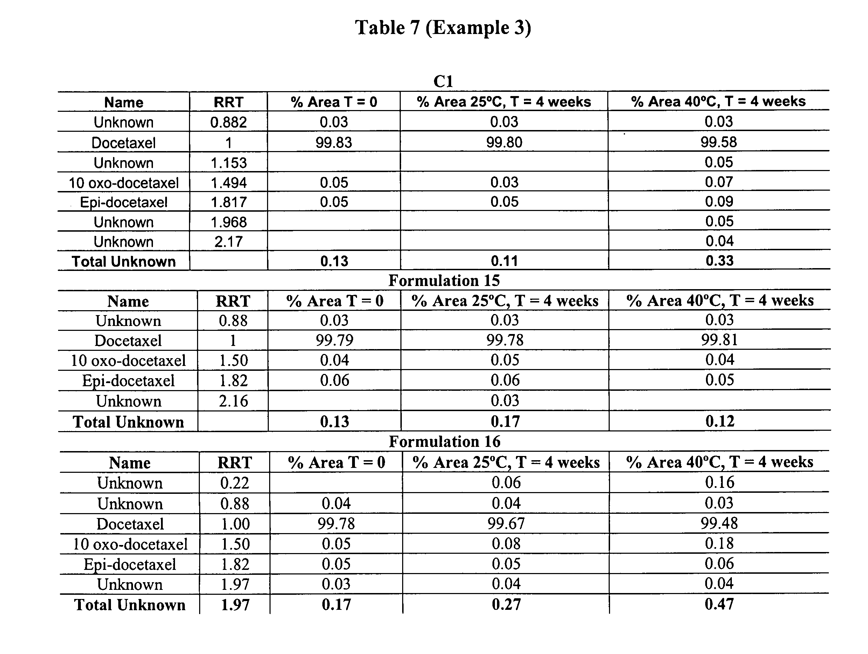

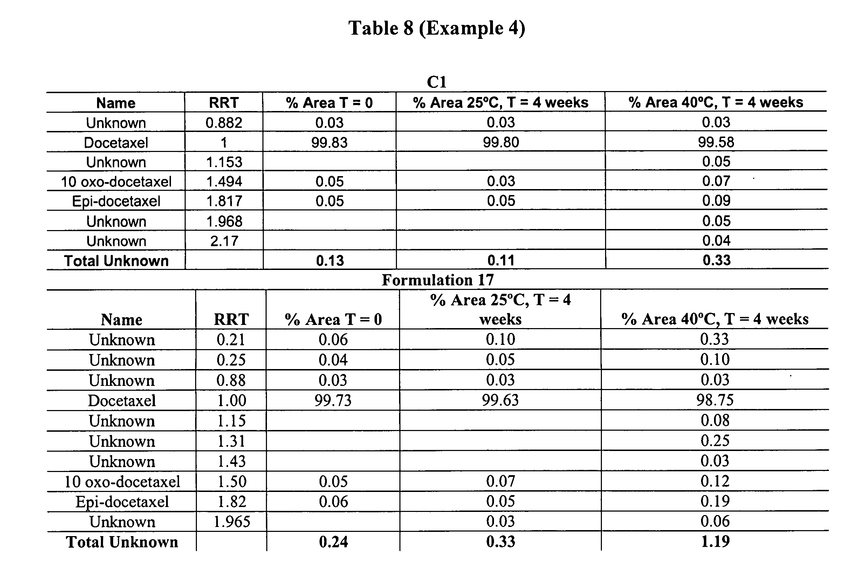

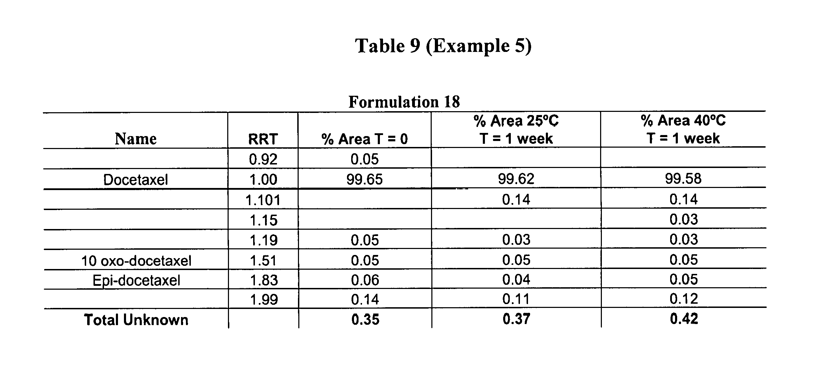

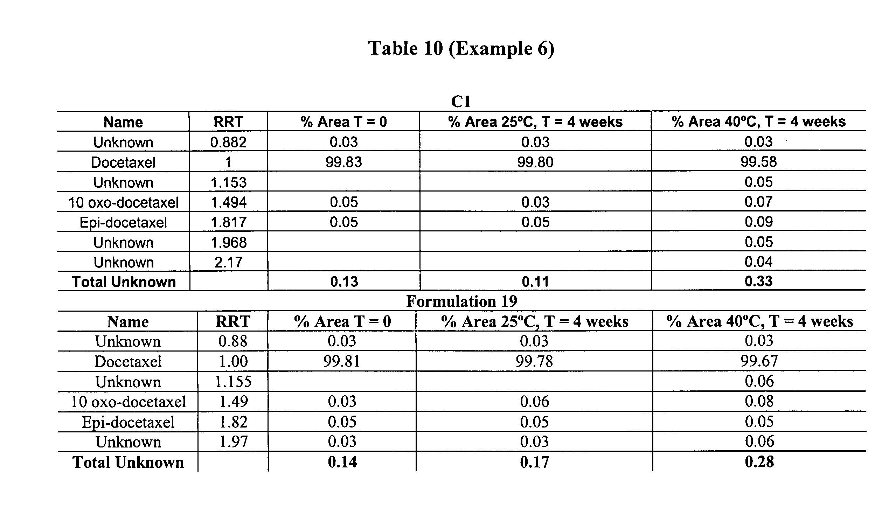

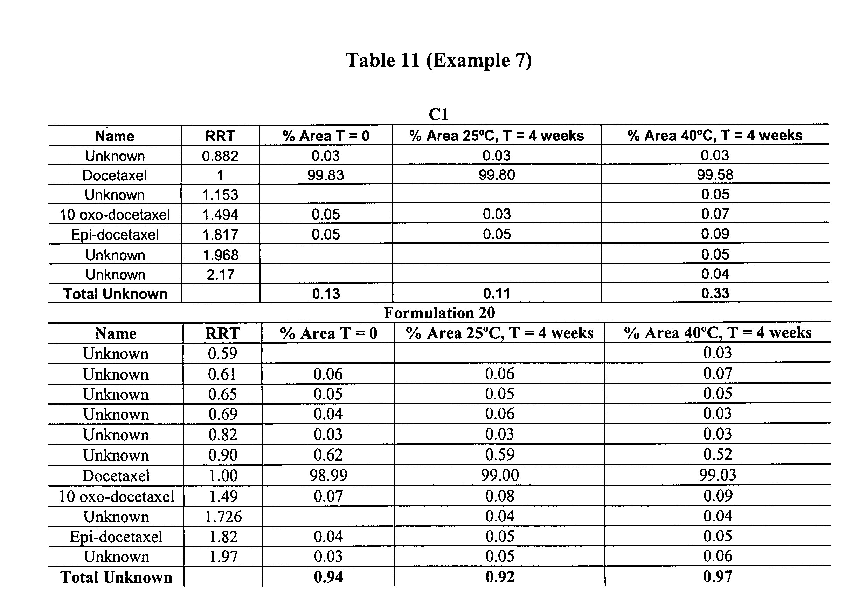

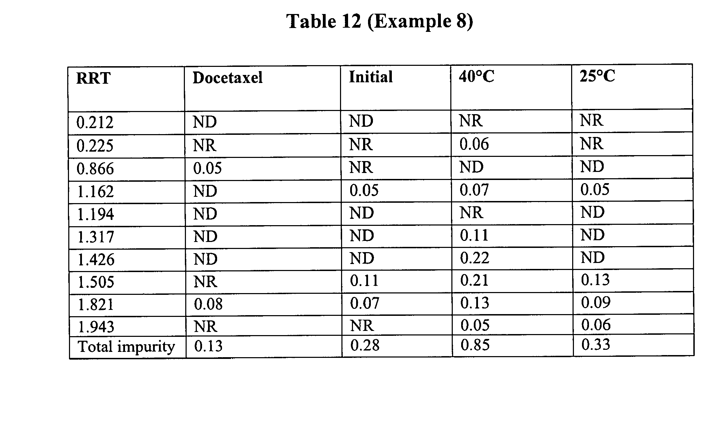

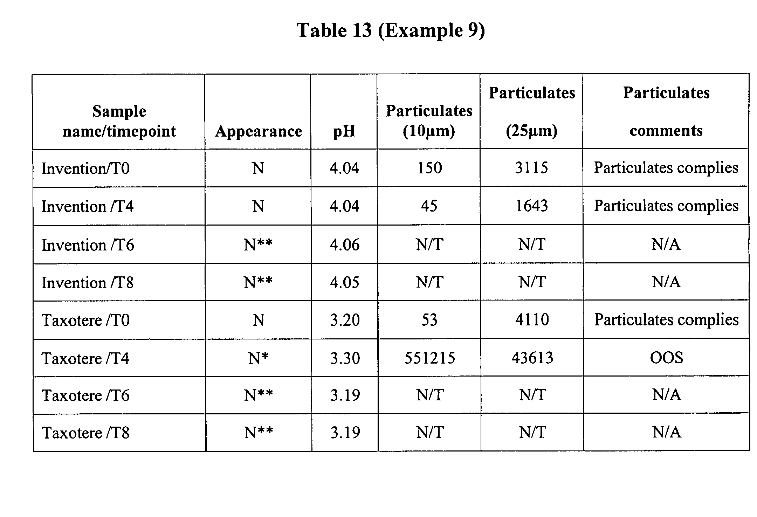

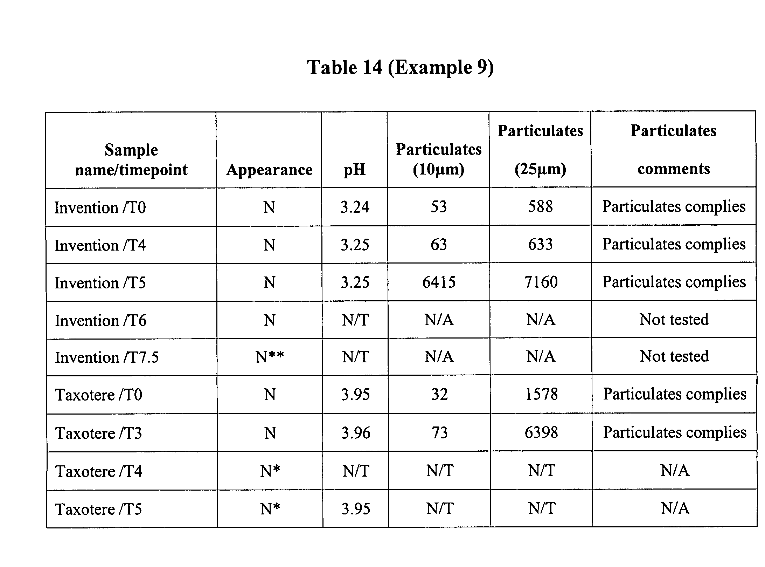

## Claims

1. A liquid pharmaceutical formulation for parenteral administration comprising:
(a) docetaxel or a pharmaceutically acceptable salt thereof;
(b) one or more glycols;
(c) an amount of one or more pharmaceutically acceptable acids sufficient to provide the formulation with a pH meter reading in the range of from 2.5 to 7;
(d) one or more alcohols; and
(e) one or more non-ionic surfactants.

2. The liquid pharmaceutical formulation according to claim 1 wherein the glycol is present in an amount in the range of from 30 to 65% v/v.

3. The liquid pharmaceutical formulation according to claim 2 wherein there is about 57% v/v of glycol.

4. The liquid pharmaceutical formulation according to claim 1 wherein the glycol is selected from the group consisting of polyethylene glycols, propylene glycol, tetra glycol and mixtures thereof.

5. The liquid pharmaceutical formulation according to claim 4 wherein the glycol is a polyethylene glycol.

6. The liquid pharmaceutical formulation according to claim 5 wherein the glycol is polyethylene glycol 300.

7. The liquid pharmaceutical formulation according to claim 1 wherein the pharmaceutically acceptable acid is an organic acid.

8. The liquid pharmaceutical formulation according to claim 7 wherein the pharmaceutically acceptable acid is selected from the group consisting of citric acid, tartaric acid, acetic acid and mixtures thereof.

9. The liquid pharmaceutical formulation according to claim 8 wherein the pharmaceutically acceptable acid is citric acid.

10. The liquid pharmaceutical formulation according to claim 9 wherein the citric acid is present at a concentration in the range of from 1.6 to 6 mg/ml.

11. The liquid pharmaceutical formulation according to claim 1 wherein the concentration of docetaxel is an amount up to 90 mg/ml.

12. The liquid pharmaceutical formulation according to claim 11 wherein the concentration of docetaxel is in the range of from 5 to 20 mg/ml.

13. The liquid pharmaceutical formulation according to claim 12 wherein the concentration of docetaxel is about 10 mg/ml.

14. The liquid pharmaceutical formulation according to claim 1 wherein the alcohol is ethanol.

15. The liquid pharmaceutical formulation according to claim 1 wherein the one or more non-ionic surfactants are selected from the group consisting of polyethoxylene sorbitan fatty acid esters, polyoxyethylene glycol esters, polyethoxylated castor oils and mixtures thereof.

16. The liquid pharmaceutical formulation according to claim 1 wherein the alcohol is ethanol and the non-ionic surfactant is one or more polysorbate 80.

17. A pharmaceutical liquid formulation for parenteral administration comprising:
(a) docetaxel at a concentration of about 10 mg/ml;
(b) polyethylene glycol 300 in an amount in the range of from 30% to 65% v/v;
(c) citric acid at a concentration in the range of from 1.6 to 6 mg/ml;
(d) polysorbate 80 in an amount in the range of from 10 to 57%; and
(e) ethanol in an amount in the range of from 10 to 50% v/v.

18. A pharmaceutical liquid formulation for parenteral administration according to claim 17 comprising:
(a) docetaxel at a concentration of about 10 mg/ml;
(b) about 57% v/v of polyethylene glycol 300;
(c) citric acid at a concentration of about 4 mg/ml;
(d) about 25% v/v of polysorbate 80; and
(e) about 23% v/v of ethanol.

19. A pharmaceutical liquid formulation for parenteral administration comprising:
(a) docetaxel or a pharmaceutically acceptable salt thereof at a concentration in the range of from 5 to 20 mg/ml;
(b) one or more pharmaceutically acceptable acids present in an amount sufficient to provide the formulation with a pH meter reading in the range of from 2.5 to 7;
(c) one or more alcohols in an amount in the range of from 10 to 55% v/v;
(d) one or more non-ionic surfactants in an amount in the range of from 10 to 50% v/v; and
(e) one or more polyethylene glycols in an amount sufficient to make up the formulation to QS 100%.

20. A pharmaceutical liquid formulation for parenteral administration according to claim 19 comprising:
(a) docetaxel at a concentration of about 10 mg/ml;
(b) citric acid at a concentration in the range of from 1.6 to 6 mg/ml;
(c) polysorbate 80 in an amount in the range of from 10 to 55%;
(d) ethanol in an amount in the range of from 10 to 50% v/v;
(e) polyethylene glycol 300 in an amount sufficient to make up the formulation to QS 100%.
